# EUROPEAN PATENT SPECIFICATION

(11) **EP 2 373 240 B1**
(45) Date of publication and mention of the grant of the patent: **29.04.2015**
(21) Application number: 09835799.9
(22) Date of filing: 22.12.2009
(51) Int. Cl.: A61B 18/02

(54) **SKIN PROTECTION FOR SUBDERMAL CRYOGENIC REMODELING FOR COSMETIC AND OTHER TREATMENTS**
HAUTSCHUTZ FÜR SUBKUTANE KRYOGENE NEUMODELLIERUNG FÜR KOSMETISCHE UND ANDERE BEHANDLUNGEN
PROTECTION CUTANÉE POUR REMODELAGE SOUS-CUTANÉ CRYOGÉNIQUE DANS LE CADRE DE TRAITEMENTS COSMÉTIQUES ET AUTRES

(30) Priority: 22.12.2008 US 139829 P
(43) Date of publication of application: 12.10.2011
(73) Proprietor: Myoscience, Inc., Redwood City, CA 94063 (US)
(72) Inventor: FOURKAS, Michael, Sunnyvale California 94087 (US); WILLIAMS, Ronald, Menlo Park California 94025 (US); GOVENJI, Punit, Los Altos Hills California 94022 (US); REYNOLDS, Byron, Gilroy California 95020 (US); OLSEN, Phillip, Cupertino California 95014 (US)
(74) Representative: Clark, Jane Anne
(86) International application number: PCT/US2009/069304
(87) International publication number: WO 2010/075448

(56) References cited:
- US-A- 4 946 460
- US-A1- 2006 173 447
- US-A1- 2007 129 714
- US-A1- 2007 129 714

## Description

### BACKGROUND OF THE INVENTION

The present invention is generally directed to medical systems particularly for cooling-induced remodeling of tissues. Embodiments of the invention include systems for applying cryogenic cooling to dermatological tissues so as to selectively remodel one or more target tissues along and/or below an exposed surface of the skin. Embodiments may be employed for a variety of cosmetic conditions, optionally by inhibiting undesirable and/or unsightly effects on the skin (such as lines, wrinkles, or cellulite dimples) or on other surrounding tissue. Other embodiments may find use for a wide range of medical indications. The remodeling of the target tissue may achieve a desired change in its behavior or composition and may temporarily inhibit contraction of a muscle so as to reduce appearance of lines and wrinkles in the face associated with contraction of the muscle.

The desire to reshape various features of the human body to either correct a deformity or merely to enhance one's appearance is common. This is evidenced by the growing volume of cosmetic surgery procedures that are performed annually.

Many procedures are intended to change the surface appearance of the skin by reducing lines and wrinkles. Some of these procedures involve injecting fillers or stimulating collagen production. More recently, pharmacologically based therapies for wrinkle alleviation and other cosmetic applications have gained in popularity.

Botulinum toxin type A (BOTOX^{®}) is an example of a pharmacologically based therapy used for cosmetic applications. It is typically injected into the facial muscles to block muscle contraction, resulting in temporary enervation or paralysis of the muscle. Once the muscle is disabled, the movement contributing to the formation of the undesirable wrinkle is temporarily eliminated. Another example of pharmaceutical cosmetic treatment is mesotherapy, where a cocktail of homeopathic medication, vitamins, and/or drugs approved for other indications is injected into the skin to deliver healing or corrective treatment to a specific area of the body. Various cocktails are intended to effect body sculpting and cellulite reduction by dissolving adipose tissue, or skin resurfacing via collagen enhancement. Development of non-pharmacologically based cosmetic treatments also continues. For example, endermology is a mechanical based therapy that utilizes vacuum suction to stretch or loosen fibrous connective tissues which are implicated in the dimpled appearance of cellulite.

While BOTOX^{®} and/or mesotherapies may temporarily reduce lines and wrinkles, reduce fat, or provide other cosmetic benefits they are not without their drawbacks, particularly the dangers associated with injection of a known toxic substance into a patient, the potential dangers of injecting unknown and/or untested cocktails, and the like. Additionally, while the effects of endermology are not known to be potentially dangerous, they are brief and only mildly effective.

In light of the above, improved medical devices, systems, and methods utilizing a cryogenic approach to treating the tissue have been proposed, particularly for treatment of wrinkles, fat, cellulite, and other cosmetic defects. These new techniques can provide an alternative visual appearance improvement mechanism which may replace and/or compliment known bioactive and other cosmetic therapies, ideally allowing patients to decrease or eliminate the injection of toxins and harmful cocktails while providing similar or improved cosmetic results. These new techniques are also promising because they may be performed percutaneously using only local or no anesthetic with minimal or no cutting of the skin, no need for suturing or other closure methods, no extensive bandaging, and limited or no bruising or other factors contributing to extended recovery or patient "down time." Additionally, cryogenic treatments are also desirable since they may be used in the treatment of other cosmetic and/or dermatological conditions (and potentially other target tissues), particularly where the treatments may be provided with greater accuracy and control, less collateral tissue injury and/or pain, and greater ease of use.

While these new cryogenic treatments are promising, careful control of temperature along the cryogenic probe is necessary in order to obtain desired results in the target treatment area as well as to avoid unwanted tissue injury in adjacent areas. Once the probe is introduced into a target treatment area, refrigerant, (also referred to as cooling fluid herein) flows through the probe and probe temperature decreases proximally along the length of the probe toward the probe hub. A proximal portion of the probe and hub is in contact with and pierces the skin. The hub may be positioned at a fixed location along the probe or may move independent to the probe allowing the probe to be inserted to variable depths while retaining skin contact. This region of the probe can become very cold which can damage the skin in the form of blistering or loss of pigmentation. Therefore, it would be desirable to provide a cryogenic device that helps control temperature along the probe thereby minimizing unwanted tissue cooling and damage. Furthermore, it would also be desirable to provide methods for controlling temperature along the cryogenic probe that would help to minimize the unwanted tissue cooling. It would also be desirable if these temperature controlling features were also cost effective, easy to manufacture and operate.

US 2007/0129714 describes a system for subdermal cryogenic remodeling comprising a self-contained probe handpiece with a handpiece housing having a size and shape suitable for supporting in the hand of a surgeon or other system operator. A cryogenic cooling fluid supply and electrical power source are found within the housing along with a circuit having a processor for controlling cooling applied by a self-contained system in response to actuation of an input.

### BRIEF SUMMARY OF THE INVENTION

The present invention is set out in the appended claims. Described herein are medical devices, systems and methods for cooling-induced remodeling of tissues. More specifically, described herein are methods and apparatus used to facilitate heating and cooling of a cryogenic device.

Described herein is a method for controlling temperature in a cryogenic device comprises providing a cryogenic device that comprises a probe and a heater element. The probe has a proximal region and a distal tissue piercing region, and the heater element is disposed along and/or adjacent the proximal region. Inserting the distal probe region through a skin surface engages the probe with a target tissue. The current temperature of the proximal probe region is measured and recorded along with the time of the measurement. The slope of a line passing through two points is determined, with the first point being defined by the current temperature and time of measurement and the second point being defined by a previous measurement of proximal region temperature and time of measurement. A treatment flag is activated and treatment start time is recorded when the calculated slope is less than a slope threshold value. When the treatment flag is activated, the proximal region of the probe is heated with the heater element. The heating parameters can vary and may include a delay time before delivering heat and varying heat applied during treatment. Heating is discontinued and the treatment flag is deactivated when elapsed treatment time exceeds a duration threshold value. The treatment time may include the time desired to maintain the probe in position after refrigerant delivery has been terminated, in particular to allow the probe to thaw prior to removal.

The method may further comprise repeating the above described steps, sometimes as long as the cryogenic device is turned on. The cryogenic device may further comprise a cooling fluid supply in fluid communication with the probe. The cooling fluid supply may comprise a canister containing from about 1 gram to about 35 grams of cooling fluid such as nitrous oxide.

The target tissue may comprise skin or muscle and the probe may comprise a needle and the step of inserting the distal probe region may comprise piercing the skin surface with the needle into the target tissue.

In some embodiments, the step of measuring comprises recording output from a thermostat such as a thermistor adjacent the proximal region. The threshold slope value may range from about -5° C per second to about -80° C per second, and more preferably ranges from about -30° C per second to about -57° C per second. The step of heating the proximal region may comprise adjusting power to the heater element based on elapsed treatment time and/or current proximal region temperature. The duration threshold may range from about 15 seconds to about 60 seconds.

The method may further comprise the step of cooling the target tissue such that the target tissue is remodeled or its function is affected and the tissue remodeling alters a shape of the skin surface. This may include cooling the target tissue to at least 0°C. Sometimes cooling the target tissue may induce necrosis in the target tissue. The method may also comprise activating the treatment flag when proximal region temperature is less than a temperature threshold value, and recording treatment start time when the treatment flag is activated. The temperature threshold value may range from about 0° C to about 10° C. Sometimes the method also includes cooling the target tissue such that the target tissue is remodeled and the tissue remodeling alters a shape of the skin surface, wherein cooling eventually overwhelms the ability of the heater element to maintain the proximal region of the probe at a higher temperature than the distal region. The method may further comprise cooling a target tissue in physiological connection with a muscle, and the cooling may temporarily inhibit contraction of the muscle so as to reduce appearance of lines and wrinkles in the face associated with contraction of the muscle.

Described herein is a method for controlling temperature in a cryogenic device comprises providing a cryogenic device comprising a probe and a heater element, the probe having a proximal region and a distal region, and wherein the heater element is disposed adjacent the proximal region. The distal probe region is engaged with the target region. Current temperature of the proximal region is measured and recorded along with the time of the measurement. Slope is determined for a line passing through a first point and a second point. The first point is defined by the current temperature and time of measurement and the second point defined by a previous measurement of proximal region temperature and time of measurement. A treatment flag is activated when the slope is less than a slope threshold value, and treatment start time is also recorded. The proximal region is heated with the heater element when the treatment flag is activated. Heating is stopped and the treatment flag is deactivated when elapsed treatment time exceeds a duration threshold value. The heater element may be in direct thermal communication with a target tissue or the thermal communication may be via the probe.

Described herein is a system for treating target tissue in a patient comprises a body having at least one cooling fluid supply path and at least one probe having a proximal portion, a distal tissue piercing portion and a lumen therebetween. The lumen is in fluid communication with the cooling fluid supply path and the at least one probe extends distally from the body and is insertable into the target tissue through a skin surface of the patient. A cooling fluid source contains a cooling fluid and is fluidly coupled with the lumen such that when cooling is initiated, cooling fluid flows in the lumen, thereby cooling the probe and any adjacent target tissue. A heater element is disposed adjacent the proximal portion and a processor system comprises a tangible computer readable medium. The tangible computer readable medium has a program configured to control the heater element thereby maintaining the proximal portion of the probe at a different temperature than the distal portion during at least a portion of the treatment.

The program may activate the heater element when a slope of a line is less than a slope threshold value, the line passing through a first point and a second point. The first point may be defined by a current temperature reading and the time of the reading, and a second point may be defined by a previous temperature reading and the time of the reading. The current temperature reading and the previous temperature readings may be adjacent the proximal region of the probe. The program may deactivate the heater when an elapsed treatment time exceeds a duration threshold value. The heater element may be movable relative to the probe. A spring element such as a coil spring or resilient elastomer may be operably coupled with the heater element so as to allow movement of the heater element relative to the probe. The probe may comprise a plurality of tissue penetrating needles.

Described herein is a system for treating target tissue in a patient comprises a body having at least one cooling fluid supply path and means for thermally engaging tissue having a proximal portion, a distal tissue piercing portion and a lumen therebetween. The lumen is in fluid communication with the cooling fluid supply path and the means for thermally engaging tissue extends distally from the body and is insertable into the target tissue through a skin surface of the patient. The system also includes means for containing a cooling fluid fluidly coupled with the lumen such that when cooling is initiated, cooling fluid flows in the lumen, thereby cooling the means for thermally engaging tissue and any adjacent target tissue and means for heating may be disposed adjacent the proximal portion. A processor system comprises a tangible computer readable medium having a program configured to control the means for heating thereby maintaining the proximal portion at a different temperature than the distal portion during at least a portion of the treatment

The program may activate the means for heating when a slope of a line is less than a slope threshold value. The line may pass through a first point and a second point, with the first point defined by a current temperature reading and the time of the reading, and the second point may be defined by a previous temperature reading and the time of the reading. The current temperature reading and the previous temperature readings may be adjacent the proximal region. The program may deactivate the means for heating when an elapsed treatment time exceeds a duration threshold value.

These and other embodiments are described in further detail in the following description related to the appended drawing figures.

### BRIEF DESCRIPTION OF THE DRAWINGS

Fig. 1A is a perspective view of a self-contained subdermal cryogenic remodeling probe and system, according to an embodiment of the invention.
Fig. 1B is a partially transparent perspective view of the self-contained probe of Fig. 1A, showing internal components of the cryogenic remodeling system and schematically illustrating replacement treatment needles for use with the disposable probe.
Fig. 2 schematically illustrates components that may be included in the treatment system.
Fig. 3 illustrates an exemplary embodiment of a needle probe having a heating element.
Figs. 3A-3B illustrate an exemplary embodiment of a floating heater element.
Figs. 3C-3D illustrate exemplary embodiments of the floating heater element.
Figs. 3E-3F illustrate exemplary embodiments of spring elements.
Fig. 4 is a flow chart illustrating an exemplary algorithm for heating the needle probe of Fig. 3.
Fig. 5 is a flow chart schematically illustrating a method for treatment using the disposable cryogenic probe and system of Fig. 1B.
Fig. 6 illustrates the cryogenic probe of Fig. 1B inserted through a patient's skin into target tissue.

### DETAILED DESCRIPTION

Described herein are medical devices, systems, and methods. Embodiments will facilitate remodeling of tissues disposed at and below the skin, optionally to treat a cosmetic defect, a lesion, a disease state, and/or so as to alter a shape of the overlying skin surface.

Among the most immediate applications may be the amelioration of lines and wrinkles, particularly by inhibiting muscular contractions which are associated with these cosmetic defects so as so improve an appearance of the patient. Rather than relying entirely on a pharmacological toxin or the like to disable muscles so as to induce temporary paralysis, many embodiments will at least in part employ cold to immobilize muscles. Advantageously, nerves, muscles, and associated tissues may be temporarily immobilized using moderately cold temperatures of 10°C to -5°C without permanently disabling the tissue structures. Using an approach similar to that employed for identifying structures associated with atrial fibrillation, a needle probe or other treatment device can be used to identify a target tissue structure in a diagnostic mode with these moderate temperatures, and the same probe (or a different probe) can also be used to provide a longer term or permanent treatment, optionally by ablating the target tissue zone and/or inducing apoptosis at temperatures from about -5°C to about -50°C. In some embodiments, apoptosis may be induced using treatment temperatures from about -1°C to about -15°C, or from about -1 °C to about -19°C, optionally so as to provide a permanent treatment that limits or avoids inflammation and mobilization of skeletal muscle satellite repair cells. Hence, the duration of the treatment efficacy of such subdermal cryogenic treatments maybe selected and controlled, with colder temperatures, longer treatment times, and/or larger volumes or selected patterns of target tissue determining the longevity of the treatment. Additional description of cryogenic cooling for treatment of cosmetic and other defects may be found in U.S. Patent Publication No. 2007/012971 4

(Attorney Docket No. 025917-000110US), filed on December 5, 2005 and entitled "Subdermal Cryogenic Remodeling of Muscle, Nerves, Connective Tissue, and/or Adipose Tissue (Fat)," and U.S. Patent Publication No. 2008/0183164 (Attorney Docket No. 025917-000120US), filed on June 28, 2007 also entitled "Subdermal Cryogenic Remodeling of Muscles, Nerves, Connective Tissue, and/or Adipose Tissue (Fat)."

In addition to cosmetic treatments of lines, wrinkles, and the like, embodiments may also find applications for treatments of subdermal adipose tissues, benign, pre-malignant lesions, malignant lesions, acne and a wide range of other dermatological conditions (including dermatological conditions for which cryogenic treatments have been proposed and additional dermatological conditions), and the like. Embodiments may also find applications for alleviation of pain, including those associated with muscle spasms as disclosed in copending U.S. Patent Application No. 12/271,013 (Attorney Docket No. 025917-00810US), filed November 14, 2008 and entitled "Pain Management Using Cryogenic Remodeling."

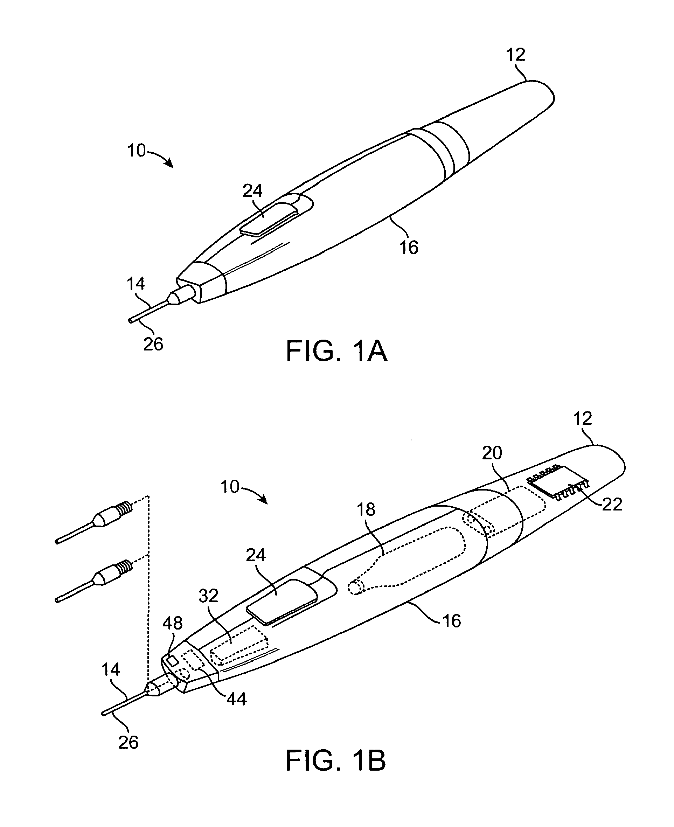

Referring now to Figs. 1A and 1B, a system for cryogenic remodeling here comprises a self-contained probe handpiece generally having a proximal end 12 and a distal end 14. A handpiece body or housing 16 has a size and ergonomic shape suitable for being grasped and supported in a surgeon's hand or other system operator. As can be seen most clearly in Fig. 1B, a cryogenic cooling fluid supply 18, a supply valve 32 and electrical power source 20 are found within housing 16, along with a circuit 22 having a processor for controlling cooling applied by self-contained system 10 in response to actuation of an input 24. Alternatively, electrical power can be applied through a cord from a remote power source. Power source 20 also supplies power to heater element 44 in order to heat the proximal region of probe 26 thereby helping to prevent unwanted skin damage, and a temperature sensor 48 adjacent the proximal region of probe 26 helps monitor probe temperature. Additional details on the heater 44 and temperature sensor 48 are described in greater detail below. When actuated, supply valve 32 controls the flow of cryogenic cooling fluid from fluid supply 18. Some embodiments may, at least in part, be manually activated, such as through the use of a manual supply valve and/or the like, so that processors, electrical power supplies, and the like may not be required.

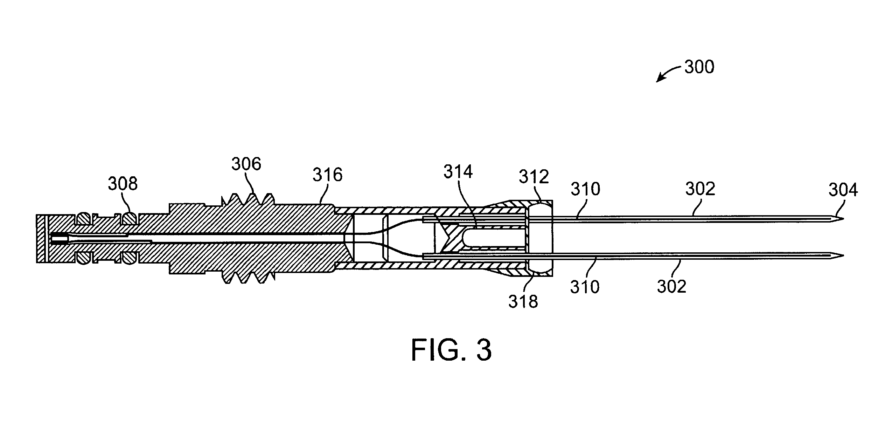

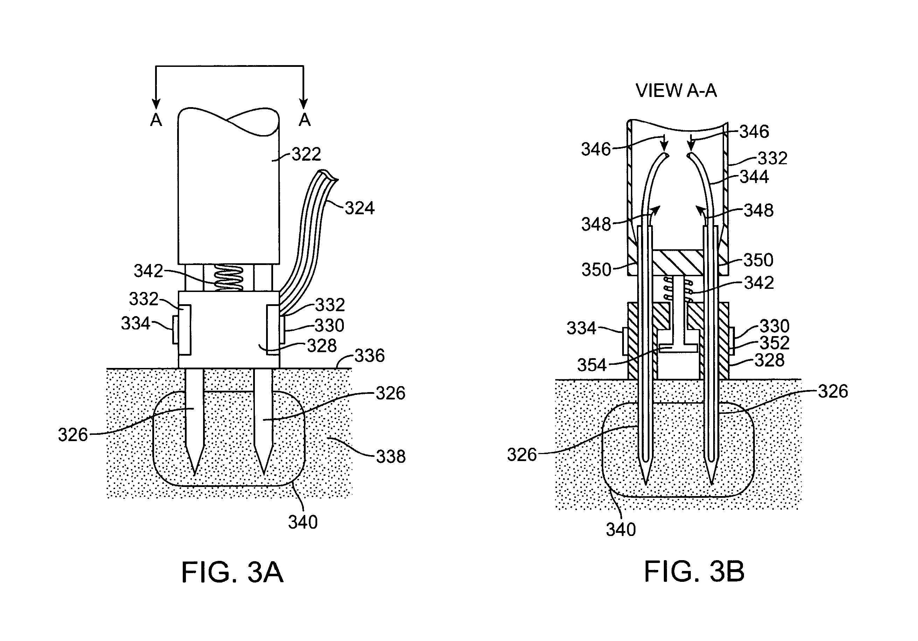

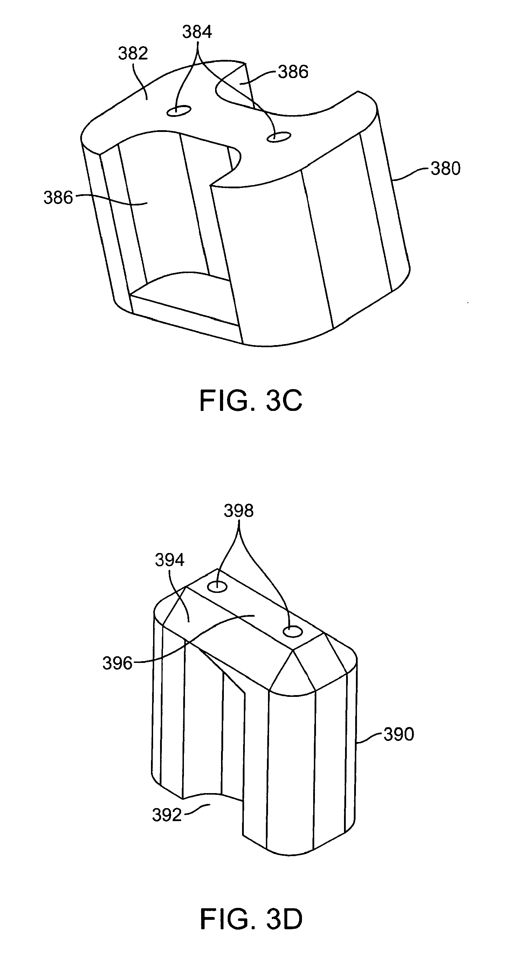

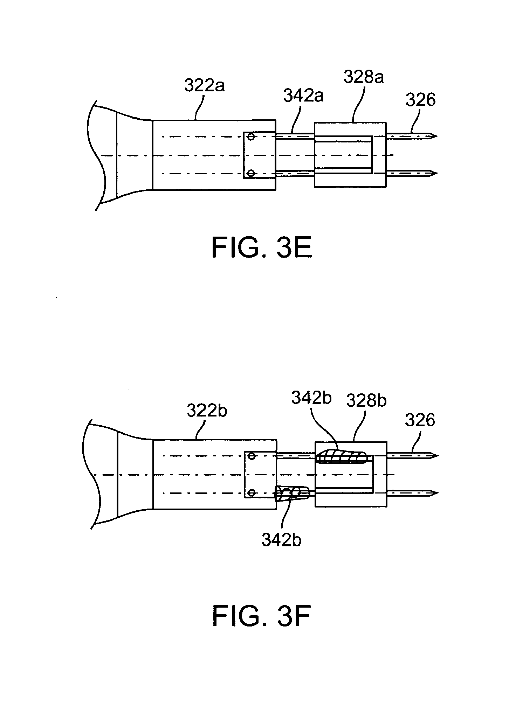

Extending distally from distal end 14 of housing 16 is a tissue-penetrating cryogenic cooling probe 26. Probe 26 is thermally coupled to a cooling fluid path extending from cooling fluid source 18, with the exemplary probe comprising a tubular body receiving at least a portion of the cooling fluid from the cooling fluid source therein. The exemplary probe 26 comprises a 30 g (gauge) needle having a sharpened distal end that is axially sealed. Probe 26 may have an axial length between distal end 14 of housing 16 and the distal end of the needle of between about 0.5 mm and 5 cm, preferably having a length from about 3 mm to about 10 mm. Such needles may comprise a stainless steel tube with an inner diameter of about 0.006 inches and an outer diameter of about 0.012 inches, while alternative probes may comprise structures having outer diameters (or other lateral cross-sectional dimensions) from about 0.006 inches to about 0.100 inches. Generally, needle probe 26 will comprise a 16 g or smaller size needle, often comprising a 20 g needle or smaller, typically comprising a 25 g or smaller needle. In some embodiments, probe 26 may comprise two or more needles arranged in a linear array, such as those disclosed in U.S. Patent Publication No. 2008/0183164 (Attorney Docket No. 025917-000120US), filed on June 28, 2007 and entitled "Subdermal Cryogenic Remodeling of Muscles, Nerves, Connective Tissue, and/or Adipose Tissue (Fat)" . Another exemplary embodiment of a probe having multiple needles is illustrated in Fig. 3, described below. Multiple needle probe configurations allow the cryogenic treatment to be applied to a larger or more specific treatment area. Other needle configurations that facilitate controlling the depth of needle penetration and insulated needle embodiments are disclosed in U.S. Patent Publication No. 2008/0200910 (Attorney Docket No. 025917-000500US), filed February 16, 2007 and entitled "Replaceable and/or Easily Removable Needle Systems for Dermal and Transdermal Cryogenic Remodeling." Multiple needle arrays may also be arrayed in alternative configurations such as a triangular or square array. Arrays may be designed to treat a particular region of tissue, or to provide a uniform treatment within a particular region, or both. In some embodiments needle 26 is releasably coupled with body 16 so that it may be replaced after use with a sharper needle (as indicated by the dotted line) or with a needle having a different configuration. In exemplary embodiments, the needle may be threaded into the body, it may be press fit into an aperture in the body or it may have a quick disconnect such as a detent mechanism or a bayonet connector for engaging the needle with the body. A quick disconnect with a check valve is advantageous since it permits decoupling of the needle from the body at any time without excessive coolant discharge. This can be a useful safety feature in the event that the device fails in operation (e.g. valve failure), allowing an operator to disengage the needle and device from a patient's tissue without exposing the patient to coolant as the system depressurizes. This feature is also advantageous because it allows an operator to easily exchange a dull needle with a sharp needle in the middle of a treatment. One of skill in the art will appreciate that other coupling mechanisms may be used.

Addressing some of the components within housing 16, the exemplary cooling fluid supply 18 comprises a canister, sometimes referred to herein as a cartridge, containing a liquid under pressure, with the liquid preferably having a boiling temperature of less than 37°C. When the fluid is thermally coupled to the tissue-penetrating probe 26, and the probe is positioned within the patient so that an outer surface of the probe is adjacent to a target tissue, the heat from the target tissue evaporates at least a portion of the liquid and the enthalpy of vaporization cools the target tissue. A supply valve 32 may be disposed along the cooling fluid flow path between canister 18 and probe 26, or along the cooling fluid path after the probe so as to limit coolant flow thereby regulating the temperature, treatment time, rate of temperature change, or other cooling characteristics. The valve will often be powered electrically via power source 20, per the direction of processor 22, but may at least in part be manually powered. The exemplary power source 20 comprises a rechargeable or single-use battery. Additional details about valve 32 are disclosed below.

The exemplary cooling fluid supply 18 comprises a single-use canister. Advantageously, the canister and cooling fluid therein may be stored and/or used at (or even above) room temperature. The canister may have a frangible seal or may be refillable, with the exemplary canister containing liquid nitrous oxide, N₂O. A variety of alternative cooling fluids might also be used, with exemplary cooling fluids including fluorocarbon refrigerants and/or carbon dioxide. The quantity of cooling fluid contained by canister 18 will typically be sufficient to treat at least a significant region of a patient, but will often be less than sufficient to treat two or more patients. An exemplary liquid N₂O canister might contain, for example, a quantity in a range from about 1 gram to about 40 grams of liquid, more preferably from about 1 gram to about 35 grams of liquid, and even more preferably from about 7 grams to about 30 grams of liquid.

Processor 22 will typically comprise a programmable electronic microprocessor embodying machine readable computer code or programming instructions for implementing one or more of the treatment methods described herein. The microprocessor will typically include or be coupled to a memory (such as a non-volatile memory, a flash memory, a read-only memory ("ROM"), a random access memory ("RAM"), or the like) storing the computer code and data to be used thereby, and/or a recording media (including a magnetic recording media such as a hard disk, a floppy disk, or the like; or an optical recording media such as a CD or DVD) may be provided. Suitable interface devices (such as digital-to-analog or analog-to-digital converters, or the like) and input/output devices (such as USB or serial I/O ports, wireless communication cards, graphical display cards, and the like) may also be provided. A wide variety of commercially available or specialized processor structures may be used in different embodiments, and suitable processors may make use of a wide variety of combinations of hardware and/or hardware/software combinations. For example, processor 22 may be integrated on a single processor board and may run a single program or may make use of a plurality of boards running a number of different program modules in a wide variety of alternative distributed data processing or code architectures.

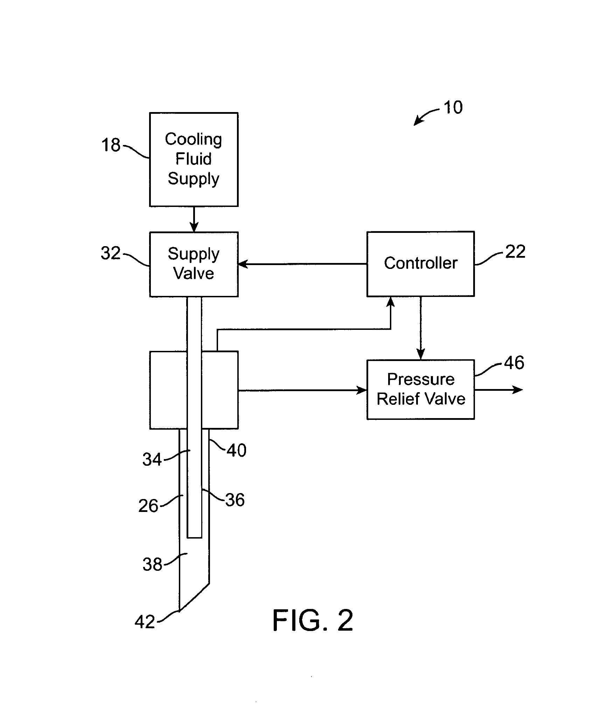

Referring now to Fig. 2, the flow of cryogenic cooling fluid from fluid supply 18 is controlled by a supply valve 32. Supply valve 32 may comprise an electrically actuated solenoid valve, a motor actuated valve or the like operating in response to control signals from controller 22, and/or may comprise a manual valve. Exemplary supply valves may comprise structures suitable for on/off valve operation, and may provide venting of the fluid source and/or the cooling fluid path downstream of the valve when cooling flow is halted so as to limit residual cryogenic fluid vaporization and cooling. Additionally, the valve may be actuated by the controller in order to modulate coolant flow to provide high rates of cooling in some instances where it is desirable to promote necrosis of tissue such as in malignant lesions and the like or slow cooling which promotes ice formation between cells rather than within cells when necrosis is not desired. More complex flow modulating valve structures might also be used in other embodiments. For example, other applicable valve embodiments are disclosed in U.S. Patent Publication No. 2008/0200910.

Still referring to Fig. 2, an optional heater (not illustrated) may be used to heat cooling fluid supply 18 so that heated cooling fluid flows through valve 32 and through a lumen 34 of a cooling fluid supply tube 36. Supply tube 36 is, at least in part, disposed within a lumen 38 of needle 26, with the supply tube extending distally from a proximal end 40 of the needle toward a distal end 42. The exemplary supply tube 36 comprises a fused silica tubular structure (not illustrated) having a polymer coating and extending in cantilever into the needle lumen 38. Supply tube 36 may have an inner lumen with an effective inner diameter of less than about 200 µm, the inner diameter often being less than about 100 µm, and typically being less than about 40 µm. Exemplary embodiments of supply tube 36 have inner lumens of between about 15 and 50 µm, such as about 30 µm. An outer diameter or size of supply tube 36 will typically be less than about 1000 µm, often being less than about 800 µm, with exemplary embodiments being between about 60 and 150 µm, such as about 90 µm or 105 µm. The tolerance of the inner lumen diameter of supply tubing 36 will preferably be relatively tight, typically being about +/- 10 µm or tighter, often being +/- 5 µm or tighter, and ideally being +/- 3 µm or tighter, as the small diameter supply tube may provide the majority of (or even substantially all of) the metering of the cooling fluid flow into needle 26. Additional details on various aspects of needle 26 along with alternative embodiments and principles of operation are disclosed in greater detail in U.S. Patent Publication No. 2008/0154254 (Attorney Docket No. 025917-000300US), filed December 21, 2006 and entitled "Dermal and Transdermal Cryogenic Microprobe Systems and Methods". U.S. Patent Publication No. 2008/0200910 (Attorney Docket No. 025917-000500US) also discloses additional details on the needle 26 along with various alternative embodiments and principles of operation.

The cooling fluid injected into lumen 38 of needle 26 will typically comprise liquid, though some gas may also be injected. At least some of the liquid vaporizes within needle 26, and the enthalpy of vaporization cools the needle and also the surrounding tissue engaged by the needle. An optional heater 44 (illustrated in Fig. 1B) may be used to heat the proximal region of the needle in order to prevent unwanted skin damage in this area, as discussed in greater detail below. Controlling a pressure of the gas/liquid mixture within needle 26 substantially controls the temperature within lumen 38, and hence the treatment temperature range of the tissue. A relatively simple mechanical pressure relief valve 46 may be used to control the pressure within the lumen of the needle, with the exemplary valve comprising a valve body such as a ball bearing, urged against a valve seat by a biasing spring. Thus, the relief valve allows better temperature control in the needle, minimizing transient temperatures. Further details on exhaust volume are disclosed in U.S. Patent Publication No. 2008/0200910.

Alternative methods to inhibit excessively low transient temperatures at the beginning of a refrigeration cycle might be employed instead of or together with the limiting of the exhaust volume. For example, the supply valve might be cycled on and off, typically by controller 22, with a timing sequence that would limit the cooling fluid flowing so that only vaporized gas reached the needle lumen (or a sufficiently limited amount of liquid to avoid excessive dropping of the needle lumen temperature). This cycling might be ended once the exhaust volume pressure was sufficient so that the refrigeration temperature would be within desired limits during steady state flow. Analytical models that may be used to estimate cooling flows are described in greater detail in U.S. Patent Publication No. 2008/0154254 (Attorney Docket No. 025917-000300US).

Turning now to Fig. 3, an exemplary embodiment of probe 300 having multiple needles 302 is described. In Fig. 3, probe housing 316 includes threads 306 that allow the probe to be threadably engaged with the housing 16 of a cryogenic device. O-rings 308 fluidly seal the probe housing 316 with the device housing 16 and prevent coolant from leaking around the interface between the two components. Probe 300 includes two distally extending needles 302, each having a sharpened, tissue penetrating tip 304. Using dual needles allows a greater area of tissue to be treated as compared with a single needle. In use, coolant flows through lumens 310 into the needles 302 thereby cooling the needles 302. Ideally, only the distal portion of the needle 302 would be cooled so that only the target tissue receives the cryogenic treatment. However, as the cooling fluid flows through the probe 316, probe temperature decreases proximally along the length of the needles 302 towards the probe hub 318. The proximal portion of needle 302 and the probe hub 318 contact skin and become very cold (e.g. -20°C to -25°C) and this can damage the skin in the form of blistering or loss of skin pigmentation. Therefore it would be desirable to ensure that the proximal portion of needle 302 and hub 318 remains warmer than the distal portion of needle 302. A proposed solution to this challenge is to include a heater element 314 that can heat the proximal portion of needle 302 and a temperature sensor 312 to monitor temperature in this region.

In the exemplary embodiment of Fig. 3, resistive heater element 314 is disposed near the needle hub 318 and near a proximal region of needle 302. The resistance of the heater element is preferably 1 Ω to 1K Q, and more preferably from 5 Ω to 50 Ω. Additionally, a temperature sensor 312 such as a thermistor or thermocouple is also disposed in the same vicinity. Thus, during a treatment as the needles cool down, the heater 314 may be turned on in order to heat the hub 318 and proximal region of needle 302, thereby preventing this portion of the device from cooling down as much as the remainder of the needle 302. The temperature sensor 312 may provide feedback to controller 22 and a feedback loop can be used to control the heater 314. The cooling power of the nitrous oxide will eventually overcome the effects of the heater, therefore the microprocessor may also be programmed with a warning light and/or an automatic shutoff time to stop the cooling treatment before skin damage occurs. An added benefit of using such a heater element is the fact that the heat helps to moderate the flow of cooling fluid into the needle 302 helping to provide more uniform coolant mass flow to the needles 302 with more uniform cooling resulting.

The embodiment of Fig. 3 illustrates a heater fixed to the probe hub. In other embodiments, the heater may float, thereby ensuring proper skin contact and proper heat transfer to the skin. For example, Fig. 3A illustrates a spring actuated floating heater and Fig. 3B illustrates a cross-section of Fig. 3A taken along the line A-A. The probe hub 322 has two tissue piercing needles 326 bonded thereto with epoxy 350 (best seen in Fig. 3B) or with other adhesives or attachment means known in the art. A conductive heater block 328 is preferably fabricated from a high thermal conductivity material, such as aluminum and has a an electrically insulated coating, such as Type III anodized coating to electrically insulate it without diminishing its heat transfer properties. The heater block 328 is heated by a resister 330 or other heating element (e.g. cartridge heater, nichrome wire, etc.) bonded thereto with a heat conductive adhesive, such as epoxy 352, and a thermistor 334 also bonded to the aluminum block with heat conductive epoxy 352 allows temperature monitoring. Other temperature sensors may also be used, such as a thermocouple. The resistor 330 and the thermistor 334 are disposed in cutouts 332 in a sidewall of the heater block 328 in order to minimize profile. The floating heater 328 linearly slides along the needles 326 and a spring 342 allows the heater block 328 to move relative to the distal end of the probe hub 322, thus the heater block maintains firm contact with the skin as the needles 326 penetrate the skin surface 336 and enter into the tissue 338. A flex circuit 324 electrically couples the resistor and thermistor with the power source and controller unit in the handpiece, and allows movement of the heater block without causing wires to tangle. The spring illustrated in this embodiment is a coil spring, however, one of skill in the art will appreciate that any compression member may be used such as a resilient member, a foam spacer, or other springlike mechanisms. In use, once the needles 326 are inserted into the tissue 338 and the needles are cooled with a steady or pulsatile flow of refrigerant, an iceball 340 will form in the tissue. However, the heater block 328 prevents the tissue from being overcooled and becoming damaged by heating the skin and heating a proximal portion of the needles, thereby preventing overcooling. Fig. 3B illustrates how refrigerant such as nitrous oxide flows into 346 the silica tubes 344 which are disposed in the needles 326 and then the refrigerant is exhausted 348 out of the needles 326. Additionally, a stopping element 354 prevents the heater block 328 from falling off the distal end of the needles 326.

In this exemplary embodiment, two needles are illustrated. One of skill in the art will appreciate that a single needle may be used, as well as three, four, five, six, or more needles may be used. When a plurality of needles are used, they may be arranged in any number of patterns. For example, a single linear array may be used, or a two dimensional or three dimensional array may be used. Examples of two dimensional arrays include any number of rows and columns of needles (e.g. a rectangular array, a square array, elliptical, circular, triangular, etc.), and examples of three dimensional arrays include those where the needle tips are at different distances from the probe hub, such as in an inverted pyramid shape.

Figs. 3C-3D illustrate exemplary embodiments of the heater block. For example, in Fig. 3C, the heater block 380 has a generally rectangular shaped body with a planar distal surface 382 for engaging skin. The body has a pair of cutouts 386 for mounting the thermistor and resistor and a pair of longitudinal channels 384 in which the needles are inserted. Fig. 3D illustrates another exemplary embodiment of a heater block 390 having a generally rectangular body with a tapered distal end 394 and a planar skin contacting surface 396. The body has one or more cutouts 392 for holding the resistor and thermistor and a pair of longitudinal channel 398 into which the needles are inserted.

Figs. 3E-3F illustrate still other exemplary embodiments of spring elements used to control movement of the heater. For example, in Fig. 3E, a resilient elastomer 342a is disposed between the heater element 328a and a distal portion of the hub 322a. The durometer of the elastomer 342a may be selected to provide desired compression and expansion characteristics for heater element 328a to slide over needles 326. Fig. 3F illustrates still another exemplary embodiment having multiple coil springs 342b disposed along different locations of the needles 326. A first spring element 342b is disposed between a distal face of the hub 322b and a proximal face of the heater element 328b. A second spring is disposed in the heater element.

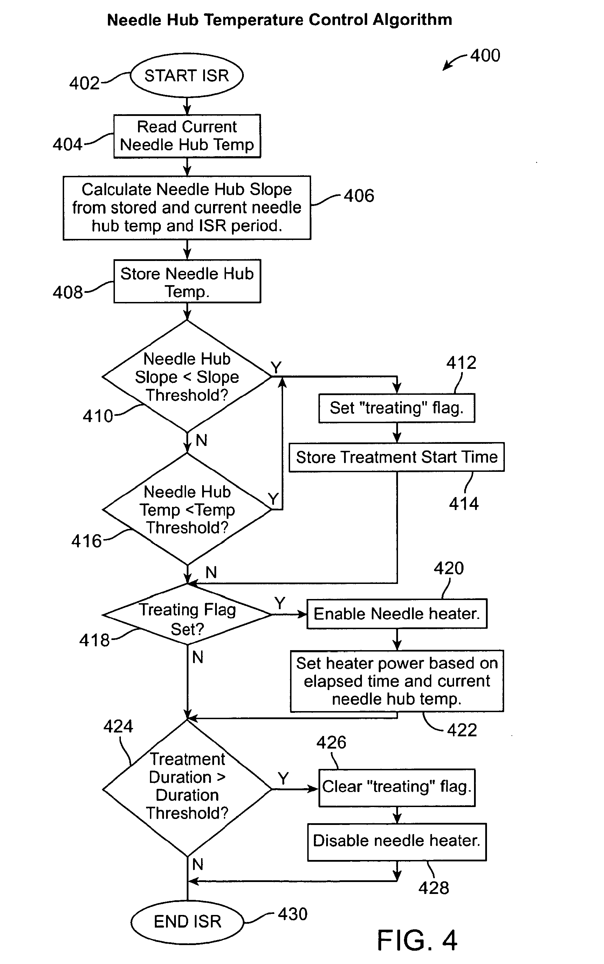

An exemplary algorithm 400 for controlling the heater element 314 is illustrated in Fig. 4. In Fig. 4, the start of the interrupt service routine (ISR) 402 begins with reading the current needle hub temperature 404 using a temperature sensor such as a thermistor or thermocouple disposed near the needle hub. The time of the measurement is also recorded. This data is fed back to controller 22 where the slope of a line connecting two points is calculated. The first point in the line is defined by the current needle hub temperature and time of its measurement and the second point consists of a previous needle hub temperature measurement and its time of measurement. Once the slope of the needle hub temperature curve has been calculated 406, it is also stored 408 along with the time and temperature data. The needle hub temperature slope is then compared with a slope threshold value 410. If the needle hub temperature slope is less than the threshold value then a treating flag is activated 412 and the treatment start time is noted and stored 414. If the needle hub slope is greater than or equal to the slope threshold value 410, an optional secondary check 416 may be used to verify that cooling has not been initiated. In step 416, absolute needle hub temperature is compared to a temperature threshold. If the hub temperature is less than the temperature threshold, then the treating flag is activated 412 and the treatment start time is recorded 414 as previously described. As an alternative, the shape of the slope could be compared to a norm, and an error flag could be activated for an out of norm condition. Such a condition could indicate the system was not heating or cooling sufficiently. The error flag could trigger an automatic stop to the treatment with an error indicator light. Identifying the potential error condition and possibly stopping the treatment, may prevent damage to the proximal tissue in the form of too much heat, or too much cooling to the tissue. The algorithm preferably uses the slope comparison as the trigger to activate the treatment flag because it is more sensitive to cooling conditions when the cryogenic device is being used rather than simply measuring absolute temperature. For example, a needle probe exposed to a cold environment would gradually cool the needle down and this could trigger the heater to turn on even though no cryogenic cooling treatment was being conducted. The slope more accurately captures rapid decreases in needle temperature as are typically seen during cryogenic treatments.

When the treatment flag is activated 418 the needle heater is enabled 420 and heater power may be adjusted based on the elapsed treatment time and current needle hub temperature 422. Thus, if more heat is required, power is increased and if less heat is required, power is decreased. Whether the treatment flag is activated or not, as an additional safety mechanism, treatment duration may be used to control the heater element 424. As mentioned above, eventually, cryogenic cooling of the needle will overcome the effects of the heater element. In that case, it would be desirable to discontinue the cooling treatment so that the proximal region of the probe does not become too cold and cause skin damage. Therefore, treatment duration is compared to a duration threshold value in step 424. If treatment duration exceeds the duration threshold then the treatment flag is cleared or deactivated 426 and the needle heater is deactivated 428. If the duration has not exceeded the duration threshold 424 then the interrupt service routine ends 430. The algorithm then begins again from the start step 402. This process continues as long as the cryogenic device is turned on.

Preferred ranges for the slope threshold value may range from about -5° C per second to about -80° C per second and more preferably range from about -30° C per second to about -57° C per second. Preferred ranges for the temperature threshold value may range from about 15° C to about 0° C, and more preferably may range from about 0° C to about 10° C. Treatment duration threshold may range from about 15 seconds to about 75 seconds and more preferably may range from about 15 seconds to about 60 seconds.

It should be appreciated that the specific steps illustrated in Fig. 4 provide a particular method of heating a cryogenic probe, according to an embodiment . Other sequences of steps may also be performed according to alternative embodiments. For example, alternative embodiments may perform the steps outlined above in a different order. Moreover, the individual steps illustrated in Fig. 4 may include multiple sub-steps that may be performed in various sequences as appropriate to the individual step. Furthermore, additional steps may be added or removed depending on the particular applications. One of ordinary skill in the art would recognize many variations, modifications, and alternatives.

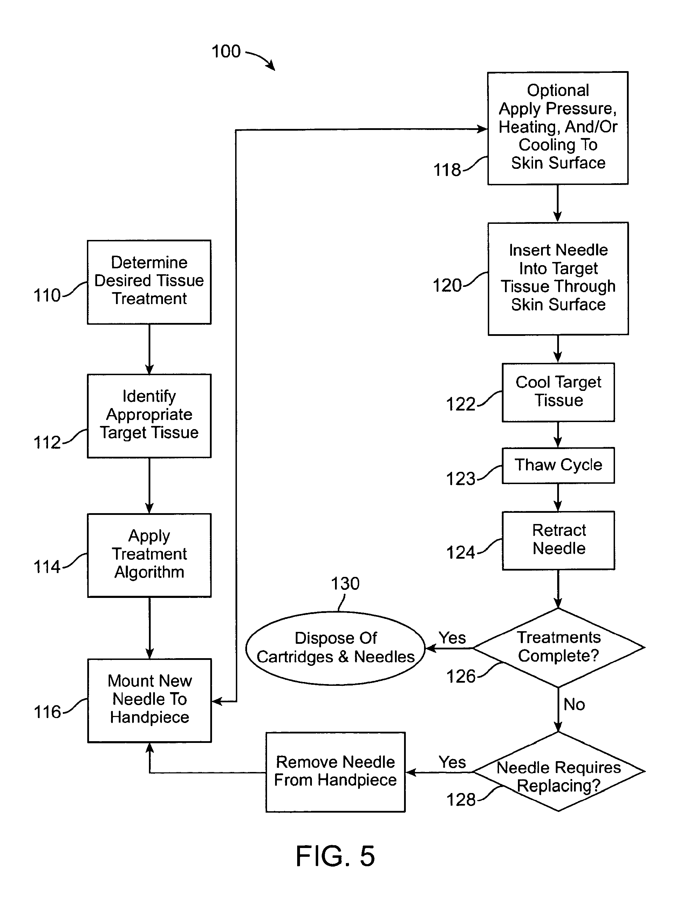

The heating algorithm may be combined with a method for treating a patient. Referring now to Fig. 5, a method 100 facilitates treating a patient using a cryogenic cooling system having a reusable or disposable handpiece either of which that can be self-contained or externally powered with replaceable needles such as those of Fig. 1B and a limited capacity battery or metered electrical supply. Method 100 generally begins with a determination 110 of the desired tissue therapy and results, such as the alleviation of specific cosmetic wrinkles of the face, the inhibition of pain from a particular site, the alleviation of unsightly skin lesions or cosmetic defects from a region of the face, or the like. Appropriate target tissues for treatment are identified 112 (such as the subdermal muscles that induce the wrinkles, a tissue that transmits the pain signal, or the lesion-inducing infected tissues), allowing a target treatment depth, target treatment temperature profile, or the like to be determined 114. The application of the treatment algorithm 114 may include the control of multiple parameters such as temperature, time, cycling, pulsing, and ramp rates for cooling or thawing of treatment areas. An appropriate needle assembly can then be mounted 116 to the handpiece, with the needle assembly optionally having a needle length, skin surface cooling chamber, needle array, and/or other components suitable for treatment of the target tissues. Simpler systems may include only a single needle type, and/or a first needle assembly mounted to the handpiece.

Pressure, heating, cooling, or combinations thereof may be applied 118 to the skin surface adjacent the needle insertion site before, during, and/or after insertion 120 and cryogenic cooling 122 of the needle and associated target tissue. Upon completion of the cryogenic cooling cycle the needles will need additional "thaw" time 123 to thaw from the internally created ice ball to allow for safe removal of the probe without physical disruption of the target tissues, which may include, but not be limited to nerves, muscles, blood vessels, or connective tissues. This thaw time can either be timed with the refrigerant valve shut-off for as short a time as possible, preferably under 15 seconds, more preferably under 5 seconds, manually or programmed into the controller to automatically shut-off the valve and then pause for a chosen time interval until there is an audible or visual notification of treatment completion.

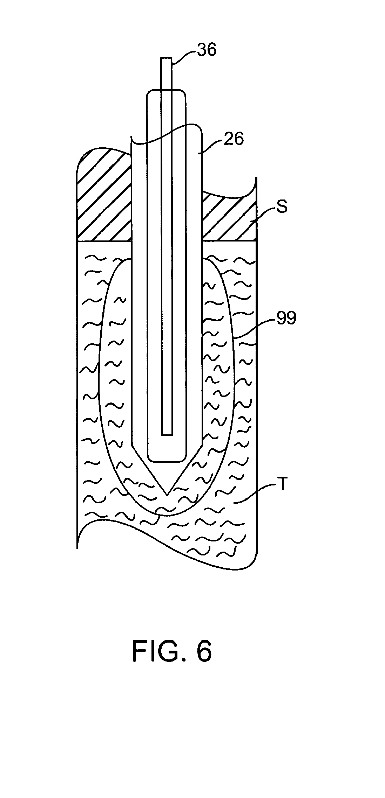

Heating of the needle may be used to prevent unwanted skin damage using the apparatus and methods previously described. The needle can then be retracted 124 from the target tissue. If the treatment is not complete 126 and the needle is not yet dull 128, pressure and/or cooling can be applied to the next needle insertion location site 118, and the additional target tissue treated. However, as small gauge needles may dull after being inserted only a few times into the skin, any needles that are dulled (or otherwise determined to be sufficiently used to warrant replacement, regardless of whether it is after a single insertion, 5 insertions, or the like) during the treatment may be replaced with a new needle 116 before the next application of pressure/cooling 118, needle insertion 120, and/or the like. Once the target tissues have been completely treated, or once the cooling supply canister included in the self-contained handpiece is depleted, the used canister and/or needles can be disposed of 130. The handpiece may optionally be discarded. Fig. 6 illustrates the needle 26 of Figs. 1A-1B and Fig. 2 after it has pierced through a patient's skin S and into the adjacent treatment tissue T. After cryogenic cooling fluid is heated and in injected into the needle 26 via supply tube 36, a region 99 of target tissue T is cooled sufficiently to effect the desired remodeling of at least a portion of the target tissue. The cooled region 99 may be controlled and shaped to treat varying tissue volumes.

A variety of target treatment temperatures, times, and cycles may be applied to differing target tissues to as to achieve the desired remodeling. For example, (as more fully described in U.S. Patent Publication Nos. 2007/0129714 and 2008/0183164.

There is a window of temperatures where apoptosis can be induced. An apoptotic effect may be temporary, long-term (lasting at least weeks, months, or years) or even permanent. While necrotic effects may be long term or even permanent, apoptosis may actually provide more long-lasting cosmetic benefits than necrosis. Apoptosis may exhibit a non-inflammatory cell death. Without inflammation, normal muscular healing processes may be inhibited. Following many muscular injuries (including many injuries involving necrosis), skeletal muscle satellite cells may be mobilized by inflammation. Without inflammation, such mobilization may be limited or avoided. Apoptotic cell death may reduce muscle mass and/or may interrupt the collagen and elastin connective chain. Temperature ranges that generate a mixture of apoptosis and necrosis may also provide long-lasting or permanent benefits. For the reduction of adipose tissue, a permanent effect may be advantageous. Surprisingly, both apoptosis and necrosis may produce long-term or even permanent results in adipose tissues, since fat cells regenerate differently than muscle cells.

While the exemplary embodiments have been described in some detail for clarity of understanding and by way of example, a number of modifications, changes, and adaptations may be implemented and/or will be obvious to those as skilled in the art. Hence, the scope of the present invention is limited solely by the independent claims.

## Claims

1. A system for treating target tissue in a patient, said system comprising:
a body (016) having at least one cooling fluid supply path (34);
engaging means (26) for thermally engaging tissue having a proximal portion, a distal tissue piercing portion (42) and a lumen (36) therebetween in fluid communication with the cooling fluid supply path (34), the engaging means extending distally from the body and being insertable into the target tissue through a skin surface of the patient;
containing means (18) for containing a cooling fluid fluidly coupled with the lumen (36) such that when cooling is initiated, cooling fluid flows in the lumen, thereby cooling the engaging means and any adjacent target tissue;
heating means (44) for heating disposed adjacent the proximal portion;
a temperature sensor (48) configured to sense the temperature of the proximal portion of the engaging means (26); and
a processor system (22) comprising a tangible computer readable medium, the tangible computer readable medium having a program configured to record the temperature of the proximal portion over time and to control the heating means (44) thereby maintaining the proximal portion at a different temperature than the distal portion during at least a portion of the treatment,
wherein the program is configured to control the heating means (44) based on a proximal portion temperature slope threshold value.

2. The system of claim 1 wherein said engaging means is at least one probe (26), said containing means (18) is a cooling fluid source containing a cooling fluid and said heating means (44) is a heater element.

3. The system of claim 1 or 2, wherein the program is configured to activate the heating means (44) when a slope of a line is less than the proximal portion temperature slope threshold value, the line passing through a first point and a second point, the first point defined by a current temperature reading and the time of the reading, and a second point defined by a previous temperature reading and the time of the reading.

4. The system of claim 3, wherein the current temperature reading and the previous temperature readings are adjacent the proximal region.

5. The system of claim 1 or 2, wherein
the program is configured to deactivate the heating means (44) when an elapsed treatment time exceeds a duration threshold value.

6. The system of claim 2, wherein the heating means (314) is movable relative to the probe.

7. The system of claim 2 wherein the system further comprises a spring element (342b) operably coupled with the heating means (314) so as to allow movement of the heating means (314) relative to the probe (300).

8. The system of claim 7, wherein the spring element (342b) comprises a resilient elastomer.

9. The system of claim 2, wherein the probe (300) comprises a plurality of tissue penetrating needles (302).

## Patentansprüche

1. System zur Behandlung von Zielgewebe in einem Patienten, wobei das genannte System Folgendes umfasst:
einen Körper (16), der mindestens einen Kühlfluidversorgungspfad (34) aufweist;
Eingriffsmittel (26) für den thermischen Eingriff in Gewebe, wobei das Eingriffsmittel Folgendes aufweist: einen proximalen Abschnitt, einen distalen Gewebedurchstechbereich (42) und ein dazwischen angeordnetes Lumen (36), das mit dem Kühlfluidversorgungspfad (34) in Fluidverbindung steht, wobei sich das Eingriffsmittel distal von dem Körper erstreckt und durch eine Hautoberfläche des Patienten in das Zielgewebe eingeführt werden kann;
Behältermittel (18) zum Aufnehmen eines Kühlfluids, das fluidtechnisch mit dem Lumen (36) gekoppelt ist, so dass, wenn die Kühlung eingeleitet wird, Kühlungfluid in dem Lumen fließt, wodurch das Eingriffsmittel und benachbartes Zielgewebe gekühlt werden;
ein Heizmittel (44) zum Erwärmen, das benachbart dem proximalen Abschnitt angeordnet ist;
einen Temperatursensor (48), der dafür konfiguriert ist, die Temperatur des proximalen Abschnitts des Eingriffsmittels (26) zu erfassen; und
ein Prozessorsystem (22), das ein physikalisches computerlesbares Medium umfasst, wobei das physikalische computerlesbare Medium ein Programm aufweist, das dafür konfiguriert ist, die Temperatur des proximalen Abschnitts über einen Zeitraum aufzuzeichnen und das Heizmittel (44) zu steuern, wodurch der proximale Abschnitt während mindestens eines Teils der Behandlung bei einer anderen Temperatur als der distale Abschnitt gehalten wird,
wobei das Programm dafür konfiguriert ist, das Heizmittel (44) basierend auf einem Temperatursteigungsschwellenwert des proximalen Abschnitts zu steuern.

2. System nach Anspruch 1, wobei die genannte Eingriffsmittel mindestens eine Sonde (26) ist, das genannte Behältermittel (18) eine Kühlfluidquelle ist, die ein Kühlfluid enthält, und das genannte Heizmittel (44) ein Heizelement ist.

3. System nach Anspruch 1 oder 2, wobei das Programm dafür konfiguriert ist, das Heizmittel (44) zu aktivieren, wenn eine Steigung einer Linie geringer ist als der Temperatursteigungsschwellenwert des proximalen Abschnitts, wobei die Linie durch einen ersten Punkt und einen zweiten Punkt verläuft, wobei der erste Punkt durch eine aktuelle Temperaturablesung und die Zeit der Ablesung definiert ist und ein zweiter Punkt durch eine vorherige Temperaturablesung und die Zeit der Ablesung definiert ist.

4. System nach Anspruch 3, wobei die aktuelle Temperaturablesung und die vorherigen Temperaturablesungen benachbart zu dem proximalen Bereichs erfolgen.

5. System nach Anspruch 1 oder 2, wobei das Programm dafür konfiguriert ist, das Heizmittel (44) zu deaktivieren, wenn eine abgelaufene Behandlungszeit einen Zeitdauerschwellenwert überschreitet.

6. System nach Anspruch 2, wobei das Heizmittel (314) relativ zu der Sonde beweglich ist.

7. System nach Anspruch 2, wobei das System ferner ein Federelement (342b) umfasst, das betriebsfähig mit den Heizmittel (314) gekoppelt ist, um eine Bewegung des Heizmittels (314) relativ zu der Sonde (300) zu ermöglichen.

8. System nach Anspruch 7, wobei das Federelement (342b) ein elastisches Elastomer umfasst.

9. System nach Anspruch 2, wobei die Sonde (300) eine Vielzahl von Gewebedurchdringungsnadeln (302) umfasst.

## Revendications

1. Système permettant de traiter un tissu cible chez un patient, ledit système comportant :
un corps (16) ayant au moins un trajet d'alimentation en fluide de refroidissement (34) ;
un moyen de mise en prise (26) permettant une mise en prise thermique du tissu ayant une partie proximale, une partie distale de perforation de tissu (42) et une lumière (36) entre celles-ci en communication fluidique avec le trajet d'alimentation en fluide de refroidissement (34), le moyen de mise en prise s'étendant de manière distale depuis le corps et étant en mesure d'être inséré dans le tissu cible au travers d'une surface de peau du patient ;
un moyen contenant (18) permettant de contenir un fluide de refroidissement accouplé de manière fluidique à la lumière (36) de telle sorte que, quand le refroidissement est initialisé, le fluide de refroidissement s'écoule dans la lumière, pour ainsi refroidir le moyen de mise en prise et tout tissu cible adjacent ;
un moyen de chauffage (44) permettant de chauffer, disposé de manière adjacente par rapport à la partie proximale ;
un capteur de température (48) configuré pour capter la température de la partie proximale du moyen de mise en prise (26) ; et
un système processeur (22) comportant un support tangible lisible par un ordinateur, le support tangible lisible par un ordinateur ayant un programme configuré pour enregistrer la température de la partie proximale au cours du temps et pour commander le moyen de chauffage (44) pour ainsi maintenir la partie proximale à une température différente par rapport à la partie distale au cours d'au moins une partie du traitement,
dans lequel le programme est configuré pour commander le moyen de chauffage (44) en fonction d'une valeur de seuil de la courbe de température de la partie proximale.

2. Système selon la revendication 1, dans lequel ledit moyen de mise en prise est au moins une sonde (26), ledit moyen contenant (18) est une source de fluide de refroidissement contenant un fluide de refroidissement et ledit moyen de chauffage (44) est un élément chauffant.

3. Système selon la revendication 1 ou la revendication 2, dans lequel le programme est configuré pour activer le moyen de chauffage (44) quand une courbe d'une ligne est inférieure à la valeur de seuil de la courbe de température de la partie proximale, la ligne traversant un premier point et un deuxième point, le premier point étant défini par un relevé de température en cours et l'heure du relevé, et un deuxième point défini par un relevé de température précédent et l'heure du relevé.

4. Système selon la revendication 3, dans lequel le relevé de température en cours et les relevés de température précédents sont adjacents par rapport à la région proximale.

5. Système selon la revendication 1 ou la revendication 2, dans lequel
le programme est configuré pour désactiver le moyen de chauffage (44) quand un temps de traitement écoulé dépasse une valeur de seuil de durée.

6. Système selon la revendication 2, dans lequel le moyen de chauffage (314) est mobile par rapport à la sonde.

7. Système selon la revendication 2, dans lequel le système comporte par ailleurs un élément de ressort (342b) accouplé fonctionnellement au moyen de chauffage (314) de manière à permettre le mouvement du moyen de chauffage (314) par rapport à la sonde (300).

8. Système selon la revendication 7, dans lequel l'élément à ressort (342b) comporte un élastomère élastique.

9. Système selon la revendication 2, dans lequel la sonde (300) comporte une pluralité d'aiguilles de pénétration dans les tissus (302).
